# EUROPEAN PATENT APPLICATION

(11) **EP 4 740 916 A1**
(43) Date of publication of application: **13.05.2026**
(21) Application number: 24836307.9
(22) Date of filing: 02.07.2024
(51) Int. Cl.: A61F 9/007, A61B 90/00

(54) **CONJUNCTIVAL MARKER AND MARKING DEVICE INCLUDING SAME**

(30) Priority: 03.07.2023 KR 20230085643
(71) Applicant: Seoul National University Hospital, Seoul 03080 (KR)
(72) Inventor: WOO, Se Joon, Seongnam-si, Gyeonggi-do 13589 (KR)
(74) Representative: V.O.
(86) International application number: PCT/KR2024/009326
(87) International publication number: WO 2025/009859

(57) **Abstract**

The present invention provides a conjunctival marker and a marking device including the same. The marking device according to one embodiment comprises a body and a marking portion coupled to the body, wherein the marking portion includes a plurality of markers in contact an eyeball, wherein the length of any one of the plurality of markers may be different from the length of at least another one of the plurality of markers.

## Description

### [Technical Field]

The present invention relates to a conjunctival marker and a marking device including the same, and more particularly, to a conjunctival marker and a marking device including the same for indicating a point at which an injection needle is inserted or withdrawn in a fixation technique for fixing a portion of an intraocular lens within the sclera.

### [CROSS-REFERENCE TO RELATED APPLICATION]

This application claims priority to and the benefit of Korean Patent Application No. 10-2023-0085643, filed on July 3, 2023, the disclosure of which is incorporated herein by reference in its entirety.

### [Background Art]

When a patient develops an ocular disease such as a cataract, a surgery is performed to insert an intraocular lens into the eyeball. When the intraocular lens is dislocated in the eyeball of a patient with an inserted intraocular lens, intrascleral intraocular lens fixation (flanged intrascleral intraocular lens fixation with double-needle technique) is required, which involves fixing a portion of the intraocular lens (e.g., the haptic) within the sclera. In the intrascleral intraocular lens fixation, positioning the intraocular lens accurately in the sclera is critical. In addition, in the intrascleral intraocular lens fixation, it is important to firmly fix the haptic (hereinafter, for convenience, collectively referred to as the intraocular lens), which is a portion of the intraocular lens into the sclera.

An intraocular lens is intrasclerally fixed by a fixation device (e.g., surgical thread). This fixation device employs an injection needle to intrasclerally secure the intraocular lens. During this process, the injection needle must be accurately inserted and withdrawn at specific points on the conjunctiva. Specifically, in the intrascleral intraocular lens fixation, the injection needle must be accurately inserted and withdrawn at six specific points on the conjunctiva.

When intrascleral intraocular lens fixation is performed after marking a plurality of specific points where the injection needle is inserted and withdrawn using measuring tools such as a protractor or ruler, the surgical time significantly increases. Furthermore, when measuring tools such as a protractor or ruler are used, the specific points where the injection needle is inserted and withdrawn may be marked inaccurately, preventing the intraocular lens from being fixed at the correct position. In addition, when marking specific points using measuring tools, the eyeball may not be fixed and may continue to move, preventing accurate marking of the desired points. This problem is exacerbated by the fact that minimizing eyeball movement is very difficult for the patient to achieve voluntarily. Furthermore, when marking specific points using measuring tools, the patient's eyeball may be damaged during contact between the measuring tools and the marking device that marks the specific points, resulting in secondary damage.

### [Disclosure]

### [Technical Problem]

One object of the present invention is to provide a marker that enables efficient surgery and a marking device including the same.

In addition, another object of the present invention is to provide a marker that allows for accurately marking a specific point on an eyeball requiring surgery and a marking device including the same.

In addition, still another object of the present invention is to provide a marker that allows for simultaneously marking specific points on an eyeball requiring surgery and a marking device including the same, minimizing the time required for surgery.

The technical problems to be solved by the present invention are not limited to the above-described problems, and problems that are not mentioned will be clearly understood by those skilled in the art from the present specification and the accompanying drawings.

### [Technical Solution]

The present invention provides a marking device including a body and a marking portion coupled to the body. The marking devices according to one embodiment may include a marking device including a body and a marking portion coupled to the body, and the marking portion may include a plurality of markers that contact the eyeball, and the length of any one of the plurality of markers may be different from the length of at least one other marker among the plurality of markers.

According to one embodiment, the marking portion may include a first marker, a second marker, and a third marker arranged to be spaced apart from each other at a predetermined distance, and the distances from the center of the body to the first marker, the second marker, and the third marker may each be different.

According to one embodiment, the distance between the first marker and the second marker may be the same as the distance between the second marker and the third marker.

According to one embodiment, the first marker may be positioned closer to the center of the body than the second marker, and the second marker may be positioned closer to the center of the body than the third marker.

According to one embodiment, the length of the first marker may be shorter than the length of the second marker, and the length of the second marker may be the same as the length of the third marker.

According to one embodiment, the length of the first marker may be shorter than the length of the second marker, and the length of the second marker may be shorter than the length of the third marker.

According to one embodiment, the body may have a ring shape, the marking portion may further include a protruding body protruding laterally from the body, the first marker may protrude downward from a lower end of the body, and the second marker and the third marker may protrude downward from a lower end of the protruding body.

According to one embodiment, the first marker and the second marker may each be positioned on a first virtual line when viewed from above, the second marker and the third marker may each be positioned on a second virtual line perpendicular to the first virtual line when viewed from above, and the first virtual line may pass through the center of the body when viewed from above.

According to one embodiment, the marking portion may include a first marking portion and a second marking portion, and each of the first marking portion and the second marking portion may include a plurality of markers, and the first marking portion and the second marking portion may have a symmetrical structure with respect to the body.

According to one embodiment, the marker may have an end formed in a round shape.

According to one embodiment, the device may be configured for use in surgeries involving markings on the eyeball, including fixation of an intraocular lens within the sclera.

### [Advantageous Effects]

According to one embodiment of the present invention, an intraocular lens can be accurately fixed within the sclera.

In addition, according to one embodiment of the present invention, an intraocular lens can be quickly fixed within the sclera.

In addition, according to one embodiment of the present invention, an intraocular lens can be fixed within the sclera while minimizing damage to the patient's eyeball.

The effects of the present invention are not limited to the above-described effects, and other effects that are not mentioned will be clearly understood by those skilled in the art from the present specification and the accompanying drawings.

### [Description of Drawings]

FIG. 1 is a cross-sectional view schematically illustrating an eyeball in which the present invention is used.
FIG. 2 is a perspective view illustrating a marking device according to one embodiment.
FIG. 3 is a lateral view illustrating a marking device according to one embodiment.
FIG. 4 is a schematic view illustrating a body and a marking portion according to one embodiment, viewed from above.
FIG. 5 is an enlarged view schematically illustrating a marking portion according to one embodiment, viewed from above.
FIG. 6 is an enlarged view schematically illustrating a marking portion according to one embodiment, viewed from the front.

### [Reference Numerals]

20: Sclera
30: Conjunctiva
40: Cornea
60: Intraocular lens
100: Eyeball
200: Marking device
210: Body
211: Connection groove
230: Connecting portion
231: First connecting portion
232: Second connecting portion
250: Gripping portion
251: Gripping protrusion
300: Marking portion
310: Protruding body
330: First marking portion
331: First marker
332: Second marker
333: Third marker
350: Second marking portion

### [Modes of the Invention]

Hereinafter, embodiments of the present invention will be described in detail with reference to the attached drawings so that those skilled in the art may easily implement the present invention. Embodiments of the present invention may be modified and implemented in various forms and are not limited to the embodiments described below. In addition, the embodiments described below are provided so that those skilled in the art may more completely explain the present invention. Therefore, the shapes of components in the drawings are exaggerated to emphasize clear explanation. In addition, when describing preferred embodiments of the present invention in detail, when it is determined that a specific description of a related known function or configuration may unnecessarily obscure the gist of the present invention, a detailed description thereof will be omitted. In addition, the same reference numerals are used throughout the drawings for parts having similar functions and operations.

When a part is said to "include" a certain component, unless specified otherwise, this means that it may further include other components, rather than exclude other components. Specifically, it is to be understood that terms such as "including" or "having" are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

Singular forms include plural forms, unless the context clearly indicates otherwise. In addition, while such terms as "first," "second," and the like may be used to describe various components, such components must not be limited to the above terms. The above terms are used only to distinguish one component from another. For example, within the scope of the present invention, a first component may be referred to as a second component, and similarly, a second component may also be referred to as a first component.

Unless otherwise defined, all terms, including technical and scientific terms used herein, have the same meaning as generally understood by one of ordinary skill in the art to which the present invention pertains. Terms, such as those defined in commonly used dictionaries, should be interpreted as having a meaning that is consistent with their meaning in the context of the relevant art and are not interpreted in an idealized or overly formal sense unless explicitly defined in the present invention.

A marking device according to one embodiment of the present invention may be used in a surgery to fix an intraocular lens into the sclera (hereinafter, intrascleral intraocular lens fixation) in a patient with a dislocated intraocular lens, such as a patient with cataract, when the intraocular lens is dislocated from the patient's eye. However, the present invention is not limited thereto, and the marking device according to one embodiment may be used in various surgeries involving marking on the eyeball to treat an ocular disease. Hereinafter, for the convenience of understanding, a case in which the marking device according to one embodiment is used in intrascleral intraocular lens fixation will be described in detail with reference to the attached drawings as an example.

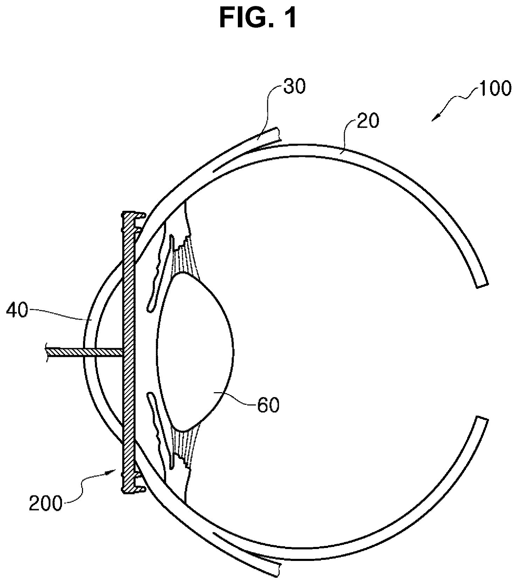

FIG. 1 is a cross-sectional view schematically illustrating an eyeball in which the present invention is used.

The eyeball illustrated in FIG. 1 is a schematic representation. In other words, the relative sizes and contact positions between the eyeball illustrated in FIG. 1 and the marking device described below are schematically illustrated. Furthermore, the relative sizes and positions of the tissues included in the eyeball illustrated in FIG. 1 may differ somewhat from those of an actual eyeball.

Referring to FIG. 1, the eyeball 100 may include a sclera 20, a conjunctiva 30, a cornea 40, and an intraocular lens 60. Intrascleral intraocular lens fixation may be performed by inserting the intraocular lens 60 into the eyeball and fixing the intraocular lens 60 within the sclera 20. In the present specification, fixing the intraocular lens 60 within the sclera 20 may be understood to mean fixing a haptic or the like, which is a part of the intraocular lens 60, within the sclera 20.

The intraocular lens 60 may be fixed within the sclera 20 by a medical thread or the like. More specifically, the medical thread or the like passes through the conjunctiva 30 to connect the intraocular lens 60 located within the eyeball 100 and the sclera 20 to each other. In order for the intraocular lens 60 to be fixed at an accurate position within the sclera 20, the position at which the injection needle (not shown) connected by the medical thread or the like is introduced and/or withdrawn into the eyeball is important. To this end, the marking device 200 described below marks in advance the position at which the injection needle (not shown) is introduced and/or withdrawn at a plurality of specific points on the conjunctiva 30. Intrascleral intraocular lens fixation is performed by introducing an injection needle (not shown) connected to a medical thread or the like into the interior of the eyeball 100 through a specific point indicated, fixing the intraocular lens 60 and sclera 20 located inside the eyeball 100 to each other, and then withdrawing the injection needle from the eyeball 100 through another specific point indicated.

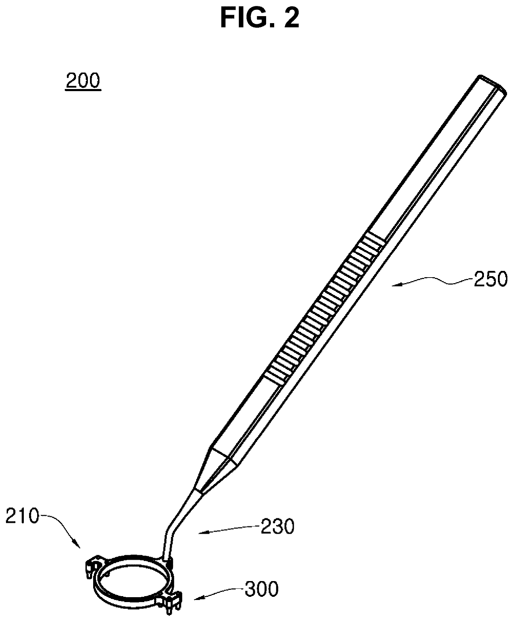

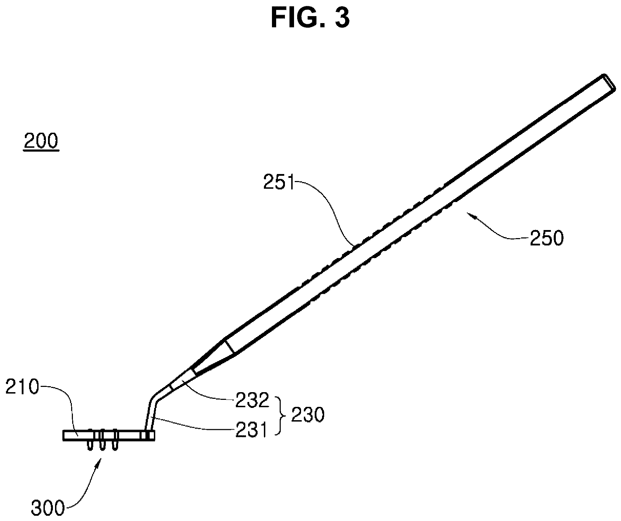

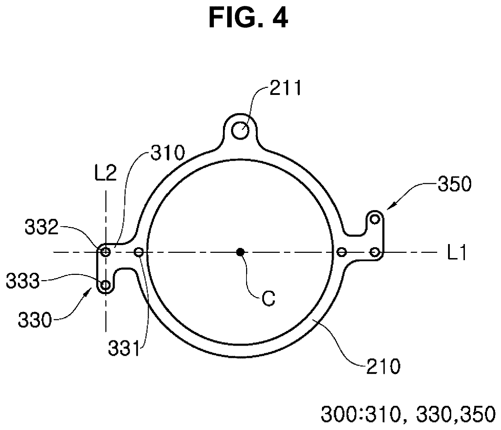

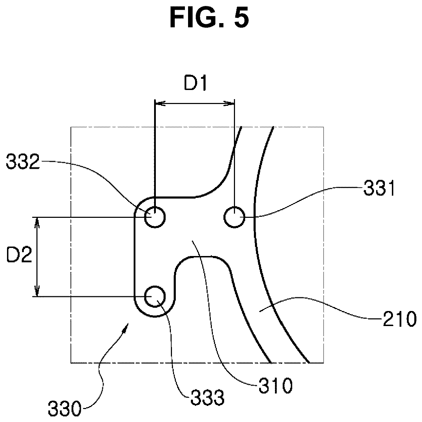

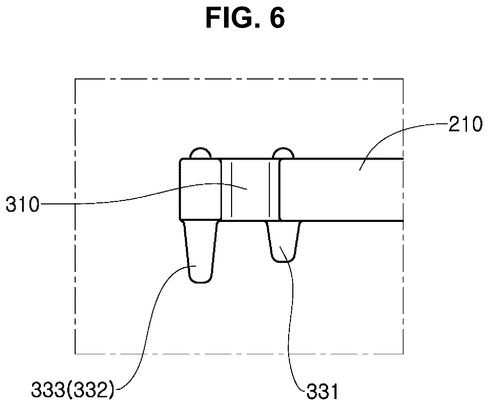

FIG. 2 is a perspective view illustrating a marking device according to one embodiment. FIG. 3 is a lateral view illustrating a marking device according to one embodiment. FIG. 4 is a schematic view illustrating a body and a marking portion according to one embodiment, viewed from above. FIG. 5 is an enlarged view schematically illustrating a marking portion according to one embodiment, viewed from above. FIG. 6 is an enlarged view schematically illustrating a marking portion according to one embodiment, viewed from the front.

Hereinafter, a marking device according to one embodiment of the present invention will be described in detail with reference to FIGS. 1 to 6.

As described above, the marking device 200 according to one embodiment may contact the eyeball 100 and mark a specific point on the eyeball 100. For example, the marking device 200 may mark a specific point on the conjunctiva 30. The mark may be a specific point where an injection needle (not shown) is introduced and/or withdrawn to secure the intraocular lens 60 within the sclera 20 in intrascleral intraocular lens fixation.

The marking device 200 according to one embodiment may include a body 210, a connecting portion 230, a gripping portion 250, and a marking portion 300.

The body 210 may be formed in an overall ring shape. For example, the body 210 may be formed in a ring shape with a portion protruding laterally. A connecting groove 211 to which a connecting portion 230, described later, is coupled may be formed in a protruding portion of the body 210. The body 210 may have a constant thickness. In addition, the material of the body 210 may include stainless steel. The diameter of the body 210 may be between approximately 11 mm and 13 mm. More specifically, the inner diameter of the body 210 may be between approximately 11 mm and 12 mm, and the outer diameter of the body 210 may be between approximately 12 mm and 13 mm. This is to efficiently perform intrascleral intraocular lens fixation by matching the diameter of the body 210 with the diameter of the intraocular lens 60 when viewed from above.

The connecting portion 230 connects the body 210 and the gripping portion 250 described below. The connecting portion 230 includes a first connecting portion 231 and a second connecting portion 232. The first connecting portion 231 may have an overall rod shape. The first connecting portion 231 is overall connected to the body 210 vertically. More specifically, one end of the first connecting portion 231 may be inserted into a connecting groove 211 formed in a protruding portion of the body 210 and connected to the body 210. The second connecting portion 232 may have an overall rod shape.

One end of the second connecting portion 232 is coupled to the first connecting portion 231. The other end of the second connecting portion 232 is coupled to the gripping portion 250. The second connecting portion 232 may have a shape in which the width thereof increases from the first connecting portion 231 toward the gripping portion 250. In addition, the longitudinal direction of the second connecting portion 232 may be inclined with respect to the longitudinal direction of the first connecting portion 231. For example, the longitudinal direction of the second connecting portion 232 may be inclined upward in a direction toward a side of the body 210. Accordingly, the connecting portion 230 may have an overall curved shape.

The gripping portion 250 may be formed in a generally rod shape. For example, a cross-section of the gripping portion 250 may have a rectangular shape. However, the present invention is not limited thereto, and a cross-section of the gripping portion 250 may be deformed into various shapes. The gripping portion 250 is coupled to the connecting portion 230. More specifically, one end of the gripping portion 250 is coupled to the second connecting portion 232. Accordingly, the gripping portion 250 is connected to the body 210 via the connecting portion 230. The longitudinal direction of the gripping portion 250 may be overall parallel to the longitudinal direction of the second connecting portion 232. In other words, the longitudinal direction of the gripping portion 250 may be inclined upward with respect to the body 210.

A gripping protrusion 251 may be formed on an upper surface and a lower surface of the gripping portion 250. The gripping protrusion 251 may be a groove. According to one embodiment, the gripping protrusion 251 may be formed only on a specific portion of the upper surface and the lower surface of the gripping portion 250. However, the present invention is not limited thereto, and the gripping protrusion 251 may be formed over the entire upper and lower surfaces of the gripping portion 250. Unlike the above, the gripping protrusion 251 may be formed only on the upper surface among the upper and lower surfaces of the gripping portion 250.

The inclined longitudinal direction of the second connecting portion 232 and the gripping portion 250 may help secure the operator's field of vision when performing intrascleral intraocular lens fixation using the marking device 200. In other words, since the connecting portion 230 and the gripping portion 250 may minimize obstruction of the operator's field of vision, intrascleral intraocular lens fixation can be performed efficiently using the marking device 200. In addition, due to the above-described gripping protrusion 251, the operator's gripping force for the marking device 200 can be improved, thereby minimizing slipping of the marking device 200 from the operator.

The above-described first connecting portion 231 and the second connecting portion 232 may be formed integrally. In addition, the above-described connecting portion 230 and the gripping portion 250 may be formed integrally. In addition, unlike the above, the connecting groove 211 may not be formed in the body 210, and the body 210, the connecting portion 230, and the gripping portion 250 may be formed integrally.

In one embodiment, the marking portion 300 may include a protruding body 310, a first marking portion 330, and a second marking portion 350.

The protruding body 310 is coupled to the body 210. In one embodiment, the protruding body 310 may be integrally coupled to the body 210. The material of the protruding body 310 may include stainless steel. The protruding body 310 protrudes laterally from the body 210. The thickness of the protruding body 310 may be the same as the thickness of the body 210. An upper surface of the protruding body 310 may be positioned at a height corresponding to an upper surface of the body 210, and a lower surface of the protruding body 310 may be positioned at a height corresponding to a lower surface of the body 210. The protruding body 310 may be formed in an overall 'L' shape. A plurality of protruding bodies 310 may be provided. For example, two protruding bodies 310 may be provided. The two protruding bodies 310 may have a symmetrical structure with respect to the center C of the body 210. For example, the two protruding bodies 310 may have a point-symmetrical structure with respect to the center C of the body 210. In addition, the two protruding bodies 310 may have a line-symmetrical structure with respect to a virtual line connecting the connecting groove 211 and the center C of the body 210. The above-described connecting groove 211 and any one protruding body 310 may be spaced apart at an interval of 90 degrees counterclockwise with respect to the center C of the body 210 when viewed from above, and the connecting groove 211 and the other protruding body 310 may be spaced apart at an interval of 90 degrees clockwise with respect to the center C of the body 210 when viewed from above. In addition, any one protruding body 310 may be symmetrically spaced apart at an interval of 180 degrees from another protruding body 310 when viewed from above. In other words, any one protruding body 310, the connecting groove 221, and the other protruding body 310 may be sequentially arranged along the circumferential direction of the body 210 when viewed from above, but may be spaced apart at an interval of 90 degrees from each other.

The first marking portion 330 and the second marking portion 350 may each include a plurality of markers. The plurality of markers may each contact the conjunctiva 30 to mark the conjunctiva 30 for intrascleral intraocular lens fixation. As described above, the first marking portion 330 and the second marking portion 350 have mostly the same or similar structures in addition to having a symmetrical structure with respect to the body 210. Therefore, the following description will be centered on the first marking portion 330.

The first marking portion 330 may include a first marker 331, a second marker 332, and a third marker 333.

Each of the markers 331, 332, and 333 may have an overall rod-shaped shape. A cross-section of each of the markers 331, 332, and 333 may have a circular shape. However, the present invention is not limited thereto, and the cross-section of each of the markers 331, 332, and 333 may of course be transformed into various shapes other than a circular shape.

An end of each of the markers 331, 332, and 333 contacts with the conjunctiva 30. The end of each of the markers 331, 332, and 333 has a round shape. When performing intrascleral intraocular lens fixation, a surgical ink or the like is applied to the end of each of the markers 331, 332, and 333, and then each of the markers 331, 332, and 333 is brought into contact the conjunctiva 30 to mark the conjunctiva 30 for intrascleral intraocular lens fixation.

The first marker 331 is coupled to the body 210. More specifically, the first marker 331 is coupled so as to protrude downward from a lower end of the body 210. According to one embodiment, the first marker 331 may be inserted into a coupling groove (not shown) formed in the body 210 and coupled to the body 210. However, the present invention is not limited thereto, and the first marker 331 may be integrally formed at a lower end of the body 210 and protrude downward from the lower end of the body 210.

The second marker 332 and the third marker 333 are each coupled to the protruding body 310. More specifically, the second marker 332 and the third marker 333 are each formed to protrude downward from the lower end of the protruding body 310. According to one embodiment, the second marker 332 and the third marker 333 may be each inserted into a coupling groove (not shown) formed in the protruding body 310 to be coupled to the protruding body 310, or may be integrally formed at the lower end of the protruding body 310. For convenience of understanding, the following description will describe an example in which the first marker 331 is inserted into a coupling groove (not shown) formed in the body 210, and the second marker 332 and the third marker 333 are each inserted into a coupling groove (not shown) formed in the protruding body 310.

The first marker 331 and the second marker 332 are each positioned on a first virtual line L1 when viewed from above. Furthermore, the second marker 332 and the third marker 333 are each positioned on a second virtual line L2 when viewed from above. The first virtual line L1 passes through each of the center C of body 210, the first marker 331, and the second marker 332, when viewed from above. The second virtual line L2 may be perpendicular to the first virtual line L1 when viewed from above. In addition, the second virtual line L2 passes through each of the second marker 332 and the third marker 333, when viewed from above.

The first marker 331 may be positioned to be spaced apart from the second marker 332 by a first distance D1 when viewed from above. For example, the first distance D1 may be 2 mm. In addition, the second marker 332 may be positioned to be spaced apart from the third marker 333 by a second distance D2 when viewed from above. For example, the second distance D2 may be 2 mm. In other words, when viewed from above, the first marker 331 and the second marker 332 may be positioned to be horizontally spaced apart by a distance of 2 mm, and the second marker 332 and the third marker 333 may be positioned vertically spaced apart by a distance of 2 mm.

In addition, the distance between the first marker 331 and the center C of the body 210 may be shorter than the distance between the second marker 332 and the center C of the body 210. In other words, the first marker 331 is positioned closer to the center C of the body 210 than the second marker 332. In addition, the distance between the second marker 332 and the center C of the body 210 may be shorter than the distance between the third marker 333 and the center C of the body 210. In other words, the second marker 332 is positioned closer to the center C of the body 210 than the third marker 333.

In one embodiment, the length of the first marker 331 is shorter than the length of the second marker 332. In addition, the length of the first marker 331 is shorter than the length of the third marker 333. In addition, the lengths of the second marker 332 and the third marker 333 may be the same. More specifically, the length of the first marker 331 protruding downward from a lower surface of the body 210 is shorter than each of the lengths of the second marker 332 and the third marker 333 protruding downward from a lower surface of the protruding body 310. For example, the difference in protruding length between the first marker 331, the second marker 332, and the third marker 333 may correspond to the difference in length between a relative center of the eyeball 100 and a relative edge of the eyeball 100 according to a curved angle of the eyeball 100.

As described above, each of the markers 331, 332, and 333 is positioned to be spaced apart by a different distance from the center C of the body 210. When the protruding lengths between the markers 331, 332, and 333 are all the same, the curved eyeball 100 may be damaged when the eyeball 100 and the markers 331, 332, and 333 come into contact each other. In particular, the first marker 331, which is closest to the center C of the body 210, may come into deep contact the surface of the eyeball 100 and cause damage to the eyeball 100. On the other hand, the third marker 333, which is positioned farthest from the center C of the body 210, may not be able to make a mark on the conjunctiva 30 because it fails to come into contact the surface of the eyeball 100. Accordingly, by making the protruding lengths between markers 331, 332, and 333 different, when performing intrascleral intraocular lens fixation, damage to the eyeball 100 caused by the markers 331, 332, and 333 due to the curved angle of the eyeball 100 can be minimized, and an accurate mark can be made on the conjunctiva 30.

Due to the rounded structure of the end of each of the above-described markers 331, 332, and 333, when the markers 331, 332, and 333 and the conjunctiva 30 come into contact each other, damage to the conjunctiva 30 can be minimized. Accordingly, when performing intrascleral intraocular lens fixation, secondary damage to the patient's eyeball due to damage can be prevented. In addition, since a plurality of markers come into contact the patient's eyeball (e.g., conjunctiva 30) at once, intrascleral intraocular lens fixation can be performed more quickly. Accordingly, the time required for the surgery can be shortened, minimizing surgical fatigue of the patient. Furthermore, since a plurality of markers are arranged to be spaced apart from each other at the accurate points where the injection needle is introduced and/or withdrawn, an accurate mark can be made at a required point with a single marking operation. Accordingly, the intraocular lens 60 can be accurately fixed at the required point within the eyeball, thereby improving the completeness of intrascleral intraocular lens fixation.

In the above-described example, the first marking portion 330 is described as including a first marker 331, a second marker 332, and a third marker 333, but is not limited thereto. For example, the first marking portion 330 may include one marker or two markers. In addition, the first marking portion 330 may further include N markers (N is a natural number greater than or equal to 4).

Although not shown, according to another embodiment of the present invention, the length of the first marker 331 is shorter than the lengths of the second marker 332 and the third marker 333. In addition, the second marker 332 may be shorter than the length of the third marker 333. More specifically, the length of the first marker 331 protruding downward from a lower surface of the body 210 is shorter than the lengths of each of the second marker 332 and the third marker 333 protruding downward from a lower surface of the protruding body 310. In addition, the length of the second marker 332 protruding downward from the lower surface of the protruding body 310 may be shorter than the length of the third marker 333 protruding downward from the lower surface of the protruding body 310. The difference in protruding length between the first marker 331 and the second marker 332 may correspond to the difference in length between a relative center of the eyeball 100 and a relative edge of the eyeball 100, which is caused by the difference in the distance of the first marker 331 and the second marker 332 from the center C of the body 210. The difference in protruding length between the second marker 332 and the third marker 333 is also the same as described above.

In addition, when the markers included in the marking portion are provided in N units (N is a natural number greater than or equal to 4), the length difference between the markers may vary depending on the distance from the center of the body 210. For example, when viewed from above, a marker that is closer to the center of the body 210 may have a shorter vertical length than a marker that is relatively farther from the center of the body 210.

According to the marking device 200 according to another embodiment of the present invention described above, damage to the eyeball 100 can be prevented more efficiently, and marking can be performed more efficiently and accurately on the conjunctiva 30, thereby further improving the completeness of intrascleral intraocular lens fixation.

The detailed description above is illustrative of the present invention. In addition, the above description describes preferred embodiments of the present invention, and the present invention may be used in various other combinations, modifications, and environments. In other words, changes or modifications are possible within the scope of the inventive concept disclosed herein, the scope equivalent to the written disclosure, and/or the scope of technology or knowledge in the art. The written embodiments illustrate the best possible state for implementing the technical spirit of the present invention, and various modifications required for specific application fields and uses of the present invention are also possible. Therefore, the detailed description of the invention above is not intended to limit the present invention to the disclosed embodiments, and the appended claims should be construed to include other embodiments.

### [Industrial Applicability]

The present invention relates to a conjunctival marker and a marking device including the same, and more particularly, to a conjunctival marker and a marking device including the same for marking a point at which an injection needle is introduced or withdrawn in a fixation technique for fixing a portion of an intraocular lens within the sclera.

## Claims

1. A marking device comprising:
a body; and
a marking portion coupled to the body,
wherein the marking portion includes a plurality of markers that contact the eyeball, and the length of any one of the plurality of markers is different from the length of at least one other marker among the plurality of markers.

2. The marking device of claim 1, wherein the marking portion includes a first marker, a second marker, and a third marker arranged to be spaced apart from each other at a predetermined distance, and the distances from the center of the body to the first marker, the second marker, and the third marker are each different.

3. The marking device of claim 2, wherein the distance between the first marker and the second marker is the same as the distance between the second marker and the third marker.

4. The marking device of claim 2, wherein the first marker is positioned closer to the center of the body than the second marker, and the second marker is positioned closer to the center of the body than the third marker.

5. The marking device of claim 4, wherein the length of the first marker is shorter than the length of the second marker, and the length of the second marker is the same as the length of the third marker.

6. The marking device of claim 4, wherein the length of the first marker is shorter than the length of the second marker, and the length of the second marker is shorter than the length of the third marker.

7. The marking device of claim 2, wherein the body has a ring shape, the marking portion further includes a protruding body protruding laterally from the body, the first marker protrudes downward from a lower end of the body, and the second marker and the third marker protrude downward from a lower end of the protruding body.

8. The marking device of claim 7, wherein the first marker and the second marker are each positioned on a first virtual line when viewed from above, the second marker and the third marker are each positioned on a second virtual line perpendicular to the first virtual line when viewed from above, and the first virtual line passes through the center of the body when viewed from above.

9. The marking device of claim 1, wherein the marking portion includes a first marking portion and a second marking portion, and each of the first marking portion and the second marking portion includes a plurality of markers, and the first marking portion and the second marking portion have a symmetrical structure with respect to the body.

10. The marking device of claim 1, wherein the marker has an end formed in a round shape.

11. The marking device of any one of claims 1 to 10, wherein the device is configured for use in surgeries involving markings on the eyeball, including fixation of an intraocular lens within the sclera.
